# EUROPÄISCHE PATENTSCHRIFT

(11) **EP 0 561 330 B1**
(45) Veröffentlichungstag und Bekanntmachung des Hinweises auf die Patenterteilung: **09.06.1999**
(21) Anmeldenummer: 93104186.7
(22) Anmeldetag: 15.03.1993
(51) Int. Cl.: A61K 38/28, A61K 31/70, A61K 38/00

(54) **Topische Arzneiformen mit Insulin**
Topical insulin preparation
Préparation topique contenant de l'insuline

(30) Priorität: 17.03.1992 DE 4208552
(43) Veröffentlichungstag der Anmeldung: 22.09.1993
(73) Patentinhaber: APL - American Pharmed Labs, Inc., Englewood Cliffs, N.J. 07632 (US)
(72) Erfinder: Liedtke, Rainer K., Dr., D-82031 Grünwald (DE)
(74) Vertreter: Tiedtke, Harro, Dipl.-Ing.

(56) Entgegenhaltungen:
- EP-A- 0 403 748
- FR-A- 2 371 926
- FR-M- 7 800
- GB-A- 2 107 985

## Beschreibung

Die Erfindung betrifft topische Arzneiformen mit Insulin zur Beeinflussung und Förderung der Zellregeneration und Wundheilung.

Es ist bekannt, daß zur Beeinflussung und Förderung der Wundheilung zahlreiche chemische Stoffe oder Stoffmischungen als Arzneimittel eingesetzt werden. Der Einsatz von Antiseptika und Antibiotika bei infektionsbedingten Wundheilungsstörungen stellt, ebenso wie der Einsatz von Hämostyptika bei initaler Wundversorgung, definierte Funktionen dar. Demgegenüber lassen sich jedoch bei den derzeit als Arzneimitteln eingesetzten Stoffen zur Förderung zelullär reparativer Wundheilungsvorgange nur in sehr begrenztem Maße Verbesserungen feststellen. Dies betrifft bereits unkomplizierte Wundheilungsverläufe, insbesondere aber die Therapie komplizierter Wundheilungen, wie z.B. postoperative oder traumatische sekundäre Wundheilungen, chronische Wundheilungsstörungen im Rahmen peripherer arterieller oder venöser Durchblutungsstörungen, allgemeine Wundheilungsstörungen im Rahmen systemischer Stoffwechselkrankheiten insbesondere bei Diabetes mellitus, durch mechanische Druckfaktoren induzierte Gewebsläsionen, wie z.B. Decubitalgeschwüre als auch chronifizierte Läsionen im Gastrointestinaltrakt wie z.B. Ulcerationen im Magen und Darmbereich.

Die derzeit eingesetzten pharmakologischen Prinzipien stützen sich dabei überwiegend auf Einzelphänomene im Wundheilungsverlauf, bei denen eine biochemisch-metabolische Insuffizienz oder einzelne Mangel-Erscheinungen als spezifische Störfaktoren angegangen werden sollen. Dementsprechend besteht auch ein sehr heterogenes Spektrum an pharmakologisch eingesetzten Stoffen. Hierzu gehören substitutive Maßnahmen wie Stoffe oder Stoffkombinationen mit Vitaminen, z.B. der Vitamine A, C, und E sowie mit Dexpanthenol (Provitamin B2), Spurenelemente, Elektrolyte, Stoffgemische mit Aminosäuren, Gelatine, granulierte Zucker etc. Als enzymatische Prinzipien werden Fibrinolytika, Streptokinase und Streptodornase, insbesondere zur Wundreinigung, eingesetzt. Als mehr unspezifische Maßnahmen, denen aber u.a. antiphlogistische, antiseptische, adstringierende oder wundtrocknende Effekte zugeschrieben werden, finden weiterhin als Stoffe oder Stoffgemische Anwendung z.B. Zinkverbindungen, Harnstoff, pflanzliche Extrakte oder Einzelstoffe aus Extrakten, wie z.B. Azulen, Hamamelis, Allantoin, lebertranhaltige Extrakte, natürliche oder künstliche Öle einschließlich Mineralölen, Perubalsam etc. Der Einsatz dieser Stoffe oder Stoffgemische erfolgt dabei teils auch wieder in Kombination mit Antiseptika oder Antibiotika.

Bei Behandlung von Läsionen und Wundheilungsstörungen im Gastrointestinaltrakt liegen zusätzliche spezifische Bedingungen vor. Therapien dortiger Ulcerationen beinhalten im oberen Gastrointestinaltrakt primär als Prinzipien Blockierungen endogener Säuresekretionen, z.B. mit H₂-Antagonisten wie Cimetidin, oder mit Protonenpumpen Inhibitoren, wie z.B. Omeprazole. Weiterhin werden zu diesem Zweck dort auch säurepuffernde Antacida, überwiegend Aluminium- und Magnesiumsalze, eingesetzt. Andere Stoffe zur Behandlung von gastrointestinalen Läsionen haben anticholinerge, damit auch spasmolytische, Wirkungen wie z.B. Pirenzepin. Eine weitere Gruppe mit wundheilungsfördernden Eigenschaften beinhaltet Stoffe mit struktureller Ähnlichkeit zu Mineralcorticoiden wie z.B. Carbenoxolol. Als ein rein topisch wirkendes mechanisch-protectives Prinzip erfolgt der Einsatz des Sacharose-Sulfats Sucralfat.

Diese Stoffe wirken primär nicht auf Prozesse direkter Gewebsregeneration auf cellulärer Ebene, sondern greifen auf verschiedenen pharmakologisch beeinflussbaren Folgestufen ein. Bezüglich einer direkt verbesserten Gewebsregeneration konnte sich bisher auch keine dieser unterschiedlichen pharmakologischen Stoffe, Stoffkombinationen oder Behandlungsprinzipien zu einer als medizinische Standardtherapie verbreiteten Behandlung etablieren. Bei gastrointestinalen Läsionen sind aber die H₂-Antagonisten, auf Grund der hohen Wirksamkeit ihres Wirkprinzips, durchaus ein Standard. Ihr Nachteil liegt in relativ hohen Rückfallraten an Ulcerationen nach Absetzen dieser Therapie wie auch einer nicht ausreichenden Wirksamkeit bei insbesondere den gefährlichen blutenden Ulcerationen (R.P. Walt et.al. 1992, The Lancet, 10, 1058).

Im Rahmen schwerwiegender Wundheilungsstörungen, insbesondere diabetisch-metabolischen Störungen, sind daher häufig auch immer noch eingreifende chirurgische Interventionen, bis hin zu Amputationen, erforderlich (e.g. I. Faris, The management of the diabetic foot, Churchill Livingstone, London, 1991; U. Knapp, Die Wunde, Thieme, Stuttgart, 1981).

Unter den zahlreichen Stoffen, deren Effekte zur Beeinflussung der Wundheilung untersucht wurden, finden sich auch für einige körpereigene Gewebefaktoren klinische Hinweise über therapeutische Effekte bei der Wundheilung, z.B. u.a. mit peptidhaltigen Extrakten aus humanen Thrombocyten (e.g. Chirurgisches Forum für experimentelle und klinische Forschung 1991, Springer, Berlin, 1991, S.10-13), wie auch für verschiedene andere Wachstumsfaktoren wie z.B. u.a. Platelet Derived Growth Factor (PDGF), Transforming Growth Factor-β (TGF-β) etc. (B. Westermark, C. Betholtz, B. Höfeld, Edits.; Growth Factors in Health and Disease, Excerpta Medica, Int. Congress Series 925, Amsterdam, 1990 ; Clinical and Experimental Approaches to Dermal and Epidermal Repair, Progress in Clin. and Biol. Research, Vol. 365, Wiley-Liss 1990; E. Müller, D. Cocchi, V. Locatelli, Advances in Growth Hormone and Growth Factor Research, Springer, Berlin/New York 1989), u.a. auch eine direkte Anwendung bei Läsionen im Gastrointestinalbereich für den Epidermal Growth Factor (EGF) (F. Halter: Regeneration following gastric injury, p.143-156, in: Morisset,J & Solomon T. E., Edits., Growth of the gastrointestinal tract, CRC Press, Boca Raton, USA, 1991 sowie für TGF-β (D.T. Cromack et al in: Clin. and Exp. Approaches to Dermal and Epidermal Repair, p.359-373 (1991) Wiley-Liss). Der biologische Status, die pharmakologische Rolle und der Wirkungsmechanismus dieser Wachstumsfaktoren ist aber noch weitgehend spekulativ oder unbekannt. Zudem ist auch weder das Zusammenspiel der einzelnen Peptidfaktoren noch deren potentielles Risiko als Einzelfaktoren Metaplasien oder Neoplasien auszulösen, bisher ausreichend bekannt (e.g., Ruski et al, J. Vasc. Surg. 14 (1991) 526; Borg et al, Lancet 1 (1989) 1268). Diese Frage gilt insbesondere für neusynthetisierte Peptide oder Peptid-Analoga. Klinischen Untersuchungen mit diesen Stoffen ergaben zudem bisher therapeutisch kontroverse Ergebnisse.

Es ist in der wissenschaftlichen Literatur beschrieben, daß das chemisch zu den Polypeptiden gehörende, den Blutzucker regulierende Hormon Insulin auch über ein zellulär-biochemisches Wirkungsspektrum verfügt (e.g. v. Bruchhausen in: Handbook of Experimental Pharmacology, Vol. XXXII/2, Springer, Berlin, S. 435-481, Curatrecasa, P., Jacobs, S., Insulin, Handbook of Exp. Pharmacology, 92 (1990), S. 289 ff., Springer, Berlin - New York), das darauf schließen lässt, daß es für eine Behandlung verletzter Gewebe geeignet erscheint. Als biochemisch-zelluläre Basiseffekte sind hierbei insbesondere aufzuführen dessen proliferativ anabole Wirkungen, d.h. eine Steigerung der Nucleinsäure- und Proteinsynthese, Wirkungen auf Kollagenstoffwechsel und Steigerung des Glukose-Transportes in Zielzellen als auch ein gesteigerter Transport verschiedener anderer Substrate. Insulin macht auf cellulärer Ebene auch Zellen kompetent für Replikationsprozesse und, in Interaktion mit anderen Wachstumsfaktoren, für eine Progression aus der G-Phase in die S-Phase (e.g. Granner, K.D. in: Harper's Biochemistry, 22th Edition (1988), Seite 556-557, Prentice-Hall).

Es ist auch klinisch bekannt, daß primär auf einem Insulinmangel beruhende Erkrankungen wie der Diabetes mellitus insgesamt schlechtere Wundheilungsverläufe und Chronifizierungen von Wunden zeigen (e.g. T. E. Bucknall, in: Postoperative Wundheilung von Organen und Organsystemen, de Gruyter, Berlin, 1990, S. 47-81). Verbesserungen der Wundheilung bei Diabetikern sind durch eine geeignete Stoffwechseleinstellung mit Insulin zu erreichen (e.g. W. Goodson et al., Surgery, Gynecology & Obstetrics 149, 600 (1979).

Spezifisch nur auf Wunden und Wundheilungsstörungen abgestellte systemische Therapien mit Insulin, insbesondere auch bei Nichtdiabetikern, finden sich aber bisher nicht. Dies könnte zudem auch zu entsprechenden systemischen Stoffwechselproblemen führen. Weiterhin ist mit einer systemischen Insulintherapie insbesondere auch keine geeignete lokale Akkumulation von Insulin in Gewebsläsionen zu erreichen, da in diesen Gewebsläsionen durch morphologische Schädigungen die lokale Perfusion eingeschränkt ist, z.B. u.a. durch Schädigungen der lokalen Mikrovasculatur, der terminalen neuronalen Fasern, durch hämostatische Effekte und entzündliche Veränderungen. Eine systemische Insulin-Therapie von Wundheilungsvorgängen ist somit, abgesehen von der als eine allgemein präoperativ-prophylaktische Maßnahme anzusehenden optimierten Einstellung des Blutzuckers bei Diabetikern, auch nicht in die Therapie eingeführt.

Demgegenüber ist aber eine lokale Anwendung von Insulin, in einer pharmazeutisch-technisch und biologisch geeigneten Anwendungsform, therapeutisch sinnvoll.

Für den Einsatz von Zellregenerationen und Wundheilungsstörungen ist die therapeutische Anwendung von Insulin in Form einer diesbezüglich auch spezifischen topischen Arzneiform bisher nicht bekanntgeworden.

Bekannt ist aber, daß in einigen experimentellen Studien am Tier, als auch bei einigen humanen Kasuistiken, schon Beschreibungen zu lokalen Anwendungen von Insulin (e.g. van Ort SR, Nurs. Res. 25 (1976) 9) erfolgten. Hier handelte es sich aber nicht um die Anwendung von für diesen Therapiezweck speziell erstellte Arzneiformen sondern nur um lokale Anwendung von Insulin in Form üblicher wässriger Injektionslösungen für Diabetiker.

Topische Anwendungen von Insulin in Form spezifischer Arzneimittel zur rein lokalen Anwendung bei Zellregeneration, Wundheilung und Wundheilungsstörungen, bei denen Insulin in einer pharmakologischen Kombination mit einem weiteren Bestandteil wie z.B. einem physiologischen Substrat, weiteren Peptidfaktoren, biologischen Cofaktoren oder auch in einer gleichzeitigen Kombination von mehreren dieser Faktoren vorliegt, sind bisher ebenfalls nicht bekannt.

Bevor sich eine spezifische lokale Therapie mit Insulin für den Zellregenerations- und Wundheilungsbereich formulieren lässt, sind grundsätzlich sehr differenzierte pharmazeutische und biologische Aspekte zur Formulierung zu berücksichtigen, die einerseits auf den spezifischen physikalisch-chemischen und biochemischen Eigenschaften des Insulinmoleküls und andererseist seinen Wechselbeziehungen mit anderen Faktoren, und zusätzlich den biologischen Abläufen bei Gewebsverletzungen beruhen. Die detaillierte Betrachtung und Korrelation dieser Aspekte ergibt, daß es technisch nicht möglich als auch therapeutisch unkritisch ist, Insulin in der unveränderten Form von Injektionslösungen wie sie bei Diabetikern eingesetzt wedren, auch für eine topische Therapie von Gewebsverletzungen anzuwenden.

Bei den pathophyiologischen Gegebenheiten eines verletzten Gewebsareals besteht ohne entsprechende pharmazeutische Maßnahmen kein Schutz des Insulins gegenüber molekularen Veränderungen wie chemischer Degradation, physikalischer Konformationsänderung, Aggregation und biologischer Inaktivierung.

Als Proteohormon muss Insulin im Wundbereich gegen enzymatische Attacken von Peptidasen geschützt werden, um nicht biologisch inaktiviert zu werden. Dies gilt sowohl gegenüber den Peptidasen endogenen Ursprungs, z.B aus Wund- und Gewebsexsudaten, aus Zellstrümmern und reaktiven Zellabbauprodukten, als auch gegenüber Peptidasen exogenen Ursprungs, z.B. aus Mikroorganismen. Eine geeignete topische Arzneiform muss daher eine diesbezüglich lokale Schutzfunktion ermöglichen. Solch ein Schutzeffekt liegt bei topischen Insulinanwendungen mittels üblicher wässriger Injektionslösungen nicht vor.

FR-M-7800 beschreibt eine lokale Anwendung von Insulin in Kombination mit insbesondere anabolen Steroiden wie Testosteron-Derivaten. Dabei wird auf die bereits bekannte Tatsache zurückgegriffen, daß diese Steroide eine Steigerung der Proteinsynthese herbeiführen, so daß das chemisch unterschiedliche Wirkprinzip von Steroiden mit dem des Peptidhormons Insulin artifiziell verknüpft wird zwecks einer damit erwarteten Erreichung einer verbesserten Wundheilung.

EP-A-0 403 748 beschreibt eine neue orale pharmazeutischtechnische Formulierung für die Anwendung unterschiedlicher Arzneistoffe mit schlechter Absorption im Magen-Darm-Trakt. U.a. wird hier auch eine orale Anwendung von Insulin für eine systemische Therapie des Diabetes mellitus beschrieben. Eine orale Anwendung wäre aber für den vorgesehenen Zweck einer topischen Anwendung sowohl unwirksam als auch technisch untauglich.

GB-A-2 107 985 beschreibt ebenfalls eine pharmazeutischtechnische Modifikation für eine Applikation von Insulin in Injektionen, und zwar als Monotherapie bei Diabetes mellitus, und somit auch keine topische Applikation. Dort wird lediglich eine bessere physikalische Stabilisierung des Insulin in üblichen Injektionslösungen für eine konventionelle systemische Monotherapie mit Injektionen bei Diabetikern beabsichtigt. Realisiert wird dies technisch mittels Zugabe von Phospholipiden in die Injektionslösung.

FR-A-2-371 926 beschreibt eine pharmazeutisch-technische Modifikation für eine systemische Insulin-Applikation bei Diabetes-Therapie sowie für eine rektale Anwendung mittels Suppositorien und beabsichtigt die Absorption von Insulin aus Zäpfchen zu steigern. Sie setzt hierzu verschiedene chemische Penetrationsförderer ein. Auch dort wird weder eine topische Anwendung im Bereich der Wundheilung beabsichtigt, noch wäre dies dazu technisch geeignet.

Um eine ausreichende Lokalwirkung zu erzeugen ist bei der Anwendung bei Gewebsschäden auch eine dauerhafte wie gleichmäßige Verteilung des Insulins erforderlich. Dies erfordert für eine spezifische topische Arzneiform eine physikalisch-chemisch definierte Spreitfähigkeit und auch eine möglichst hohe Präferenz zur Adsorption oder Adhäsion an geschädigtes Gewebe. Dies gilt insbesondere auch für topische Anwendungen im Gastrointestinalbereich. Auch diese Voraussetzungen werden mit üblichen wässrigen Injektionslösungen nicht erreicht.

Eine spezifisch geeignete topische Anwendungsform muss weiterhin den biochemischen Gegebenheiten bei Gewebsschäden Rechnung tragen, z.B. u.a. dem lokalen pH-Wert, dem lokalen osmotischem Druck, anatomischen und topographischen Besonderheiten wie der Anwesenheit von Wundexsudaten und potentiellen Infektionen etc. Auch hier zeigen wässrige Lösungen schon rasche pH-Verschiebungen auf äußere Einflüsse, was wiederum zu Änderungen der biologischen Aktivität von Insulin führt. Osmotische Einflüsse bewirken konvektive und diffusionbedingte Transportänderungen von Substraten und Cofaktoren im Wundbereich, somit gleichfalls erhebliche Einflüsse auf die Regenerationsverläufe.

Für eine lokale Therapie zur Zellregeneration und Wundheilung ist auch eine spezifische, pharmakodynamisch geeignete, lokale Dosierung erforderlich. So liegt der Dosisbereich für Insulin zur Auslösung cellulärer mitogener oder proliferative Effekte deutlich niedriger (e.g. Conover, A. et. al., Horm. Metabol. Res. 21 (1989) 59-63), ist somit deutlich unterschiedlich zu den Dosen, die zur systemisch antidiabetischen Therapie eingesetzt werden.

Weiterhin muss die Lokaltherapie auch über entsprechende lokale Dosiergenauigkeit und Anwendungsfertigkeit verfügen. Sie darf auch gegenüber üblichen anderen Insulin-Anwendungen keine Verwechsungsmöglichkeit beinhalten.

Patienten mit chronischen Wundheilungsstörungen zeigen oft überhöhte lokale Empfindlichkeit gegenüber Wirkstoffen und Begleitstoffen. Eine topische Anwendungsform bei Zellregeneration und Wundheilung muss daher auch über eine entsprechende lokale Verträglichkeit verfügen. Insulin-Injektionslösungen besitzen oft höhere Konzentrationen an Konservantien, die in Wundbereichen lokale Irritationen und Allergien auslösen können.

Im biologisch komplexen Vorgang der Gewebsregeneration ist es auch nicht ausreichend Insulin nur als ein singuläres Wirkprinzip zu betrachten. Der auf eine erheblich erhöhte Syntheseleistung umgestellte zelluläre Metabolismus im geschädigten Gewebe erfordert in dieser Phase sowohl biologische Effektoren, die eine biochemisch motorische Rolle spielen, wie auch entsprechende Substrate, die sowohl für den Intermediärstoffwechsel als auch den morphologischstrukturellen Wiederaufbau der Zelle benötigt werden. Da ein Basalprozess von Insulin die Erhöhung des cellulären Glukosetransfers ist, kann dies zu auch zu rascherer lokaler Konsumption dieser Substratreserve führen. In weiterem Verlauf kann daher eine alleinige Insulin-Gabe gegebenfalls wirkungslos werden, wenn nicht mehr ausreichendes Substrat zur Verfügung steht. Somit ist es therapeutisch auch sinnvoll, eine topische Insulin Arzneiform zusätzlich mit seinen biologischen Substraten oder auch weiteren Cofaktoren zu versehen. Hierdurch können biochemische Lokalwirkungen des Insulins direkt oder indirekt synergistisch unterstützt und aufrechterhalten werden.
Weiterhin sind dies auch andere Gewebsfaktoren, wie Wachstumsfaktoren und Peptide, deren Effekte erst im Zusammenhang mit Effekten von Insulin biochemisch zum tragen kommen.

Der Erfindung liegt die Aufgabe zugrunde, die Wundheilung und den Wundheilungsverlauf zu verbessern, insbesondere auch bei chronischen und verzögerten Wundheilungsstörungen im Rahmen metabolischer oder perfusionsbedingter Störungen.

Diese Aufgabe wird erfindungsmäßig durch die Bereitstellung von topischen Arzneiformen gemäß Anspruch 1 gelöst. In deisen topischen Arzneiformen sind Insulin, entweder als Rinder-Insulin, Schweine-Insulin oder Human-Insulin und Glukose in für lokale Anwendungszwecke pharmakologisch wirksamen Konzentrationen in pharmazeutisch für topische Anwendung geeigneten Arzneiformen, vorzugsweise einer Emulsion, Suspension. Creme, Salbe, Lösung, Schüttelmixtur, einem Gel, Puder, Granulat oder einem Pflastersystem enthalten, wobei diese Arzneiformen als zusätzliche Bestandteile Einfachzucker, Elektrolyte, andere Peptidstoffe als Insulin, Proteasehemmstoffe, Antioxidantien, Emulgatoren und Konservantien enthalten können und diese zusätzlichen Bestandteile einzeln oder in verschiedenen Kombinationen vorliegen können.

Ein weiterer Gegenstand der Erfindung besteht in der Verwendung einer Kombination von Insulin und Glukose zur Herstellung von topischen Arzneiformen für therapeutische Anwendungen gemäß Anspruch 7.

Um die Wirksamkeit der topischen Arzneiformen mit Insulin weiter zu steigern, ist, in einer weiteren Ausbildung der Erfindung, die Arzneiform eine Emulsion, bei der Insulin in der wässrigen Phase enthalten ist und die Ölphase vorzugsweise aus Tricylglyceriden und Partialglyceriden gesättigter Fettsäuren mit Kettenlängen von 6-18 Kohlenstoffatomen oder einer Kombination dieser Acylglyceride mit Phospholipiden, insbesondere Lecithin, besteht.

Um die Wirksamkeit der topischen Arzneiformen mit Insulin weiter zu steigern, sind, in einer weiteren Ausbildung der Erfindung, bei den topischen Arzneiformen Stoffe als Vehikel eingesetzt, die mit besonderen Adsorptionsfähigkeiten zur Anlagerung an verletzte Zellen und Gewebe ausgestattet sind, ein derartiger Stoff insbesondere Sucralfat, das basische Aluminiumsalz von Saccharose-Octahydrogensulfat ist, und diese Stoffe sowohl allein oder auch in Kombination mit anderen Vehikelstoffen vorliegen.

Um die Wirksamkeit der topischen Arzneiformen mit Insulin weiter Zu steigern, enthalten, in einer weiteren Ausbildung der Erfindung, die Vehikel der topischen Arzneiformen polymere Stoffe, wobei diese Stoffe insbesondere Polysaccharide und Mucopoiysaccharide` Polypeptide und Proteine, Lipopolysaccharide, Glykoproteine und Lipoproteine sind, und diese in Kombinationen oder in Kombinationen mit anderen Vehikelstoffen vorliegen können.

Um die Wirksamkeit der topischen Arzneiformen mit Insulin zu verbessern, enthält die Arzneiform, in einer weiteren Ausbildung der Erfindung, weitere Einfachzucker, als zusätzlichen cofaktor vorzugsweise die Elektrolyte Natrium, Kalium, Magnesium, Calcium und Zink oder deren Salze als Einzelstoffe oder in Kombination.

Um die Wirksamkeit der topischen Arzneiformen mit Insulin weiter zu steigern, sind, in einer weiteren Ausbildung der Erfindung, als zur Stabilisierung eingesetzte Emulgatoren vorzugsweise Lecithine, Glycerinester, Palmitinstearylalkohole, Polyethylenglykole oder deren Gemische.

Um die Wirksamkeit der topischen Arzneiformen mit Insulin weiter zu verbessern, sind, in einer weiteren Ausbildung der Erfindung, die zusätzlich und in Kombination mit Insulin weiter enthaltenen Peptide und Proteine, insbesondere auch Platelet Derived Growth Factor (PDGF), Platelet Derived Angiogenesis Factor (PDAF), Epidermal Growth factor (EGF), Platelet Derived Endothelial Cell Growth Factor (PDECGF), Platelet Factor 4 (PG4), Transforming Growth Factor-β (TGF-β), Nerve Growth Factor (NGF), Insulin Like Growth Factor I und II (IGF I, IGF II), Growth Hormone (hGF), Interleukin 2 (Il-2), Heparin Binding Growth Factor (HBGF I), Human Growth Hormone Release Factor (hGRF), Fibrocyte Growth Factor (FGF), Colony Stimulating Factor (CSF), Streptodornase, Streptokinase, sowohl als zusätzliche Einzelstoffe oder in verschiedenen Kombinationen.

Um die Wirksamkeit der topischen Arzneiformen mit Insulin weiter zu verbessern, sind, in einer weiteren Ausbildung der Erfindung, in der Arzneiform als Antioxidans, vorzugsweise Ascorbinsäure oder Tocopherol, auch als ihre Salze und Ester enthalten.

Um die Wirksamkeit der topischen Arzneiformen mit Insulin weiter zu verbessern, ist, in einer weiteren Ausbildung der Erfindung, als Proteaseinhibitor vorzugsweise Aprotinin, Soja Bean Trypsin Inhibitor (SBTI), Lima Bean Trypsin Inhibitor (LBTI) oder ein Gemisch dieser Proteasehemmer, enthalten.

Um den therapeutischen Einsatz der topischen Arzneiformen mit Insulin geeignet zu nutzen, ist in einer weiteren Ausbildung der Erfindung, die therapeutische Anwendung nicht auf spezielle Gewebe mit postoperativen, traumatischen, physikalischen oder metabolisch bedingten Wunden und Wundheilungsstörungen beschränkt sondern bezieht ein topische therapeutische Anwendung bei allen topisch behandelbaren Geweben und Organen ein, ausdrücklich somit die therapeutischen Anwendungen bei physikalischen oder biochemischen Gewebsläsionen im Bereich peripherer und zentraler Nerven, im Knochengerüst, der Gelenke und Knorpelstrukturen sowie im Bereich aller Schleimhäute, insbesondere auch der Schleimhäute im Gastrointestinaltrakt und im Genitalbereich.

Um die Anwendung der topischen Arzneiformen mit Insulin technisch zu verbessern, sind, in einer weiteren Ausbildung der Erfindung, die topischen Arzneiformen in spezielle Behältnissen eingebracht, aus denen eine direkte als auch dosierbare Applikation in das jeweils zu behandelnde Gewebsgebiet erfolgen kann.

Um die Anwendung der topischen Arzneiformen mit Insulin weiter zu verbessern, ist, in einer weiteren Ausbildung der Erfindung die topische Arzneiform in einen aus physiologisch verträglichem Material bestehenden festen oder elastischen Körper unterschiedlicher Gestalt eingebracht, wobei dieser Körper insgesamt in das zu behandelnde Gewebsareal eingelegt wird und aus diesem die topischen Arzneiformen kontrolliert in das zu behandelnde Wundgebiet freigesetzt werden.

Die mit der Erfindung erzielten Vorteile bestehen insbesondere darin, daß mit den topischen Arzneiformen eine gezielte Intensivierung zellulärer Insulineffekte erfolgt. Hierbei wird vor allem die im geschädigten Gewebe vorliegende lokale negative Energiebilanz verbessert, die auf der Diskrepanz von cellulär erhöhtem Energiebedarf für Syntheseleistungen bei gleichzeitigem lokalem Mangel an energetisch verwertetem Substrat besteht. Es kommt durch die topische Insulin-Arzneiform zu proliferativen Effekten wie Steigerung von lokaler Proteinbiosynthese und Bindegewebsstoffwechsel. Dies stellt eine substanzielle Basis für die reparativen Vorgänge im geschädigten Gewebe dar und erfolgt weitestgehend ohne unspezifischen Einbezug des Gesamtorganismus.

Eine Verbesserung der Energiebilanz durch den lokal intensivierten Glukosetransfer der Zellen des Wundgebietes, bei denen seinerseits ein Transportmangel, durch lokale Hämostase, lokale Gefäßschäden und Entzündungsphänomene vorliegt, kann zusätzlich durch ein gleichzeitiges Angebot der als Substrat dienenden Glukose im Trägervehikel weiter verbessert werden, da Gewebsschädigungen energetisch einige Analogien zeigen, die mit einem lokalen Diabetes-Syndrom vergleichbar sind.

Die zusätzliche Gegenwart von Elektrolyten, die als enzymatische Cofaktoren dienen, führt dabei zu einer unspezifischen Verbesserung lokaler enzymatischer Aktivitäten wobei z.B. Kalium die Insulinsensitivität steigern kann und Magnesium als Cofaktor bei der gesteigerten Proteinbiosynthese dient.

Pharmazeutisch-technisch wird, z.B. auch durch Wahl einer geeignet stabilisierten Emulsion, beispielsweise auch einer einer Mikroemulsion, das enthaltene Insulin gleichförmig über das gesamte Gewebsareal gespreitet. Die Lipid-Phasen, insbesondere auch die Anwendung von Partialgylceriden und Phospholipiden, zeichnet sich dabei durch gute Gewebsadsorption und ausgezeichnete biologische Verträglichkeit bzw. praktische Atoxizität aus. Insulin tritt aus den topischen Arzneiformen successiv aus und ermöglicht somit geeignete lokale Dosierungen mit ausreichender Wirkdauer der Einzeldosen. Weiterhin stellen die Lipidphasen eine effektive Schutzbarriere gegen endogene oder mikrobielle Proteasen dar, die im Wundbereich häufig auftreten.

Ein Verwendung von geeigneten Vehikelstoffen, z.B. von Sucralfat, die eine erhöhte Anlagerung und Adhäsivität an Zellareale mit Oberflächenläsionen aufweisen, führen zu einem verbessertem Targeting der Arzneiformen. Solche Effekte sind insbesondere bei topischen Anwendungen im Gastrointestinalbereich von Bedeutung, bei denen eine perorale Arzneiformulierung erforderlich ist. So finden sich z.B. auch für Sucralfat nach peroraler Applikation eine erhöhte Akkumulation an ulceriertem Gewebe gegenüber unverletztem Gewebe (Sasaki, H. e.l., Bindung von Sucralfat an Duodenalulcera bei Menschen, in Marks,I.H. (Edit.), Sucralfat, Edition Medizin, Weinheim, 1983, S. 9-12).

Ein Schutzeffekt gegenüber Peptidasen lässt sich auch durch die Zugabe geeigneter Protease-Hemmstoffe in die Arzneiform weiter steigern. Oxidative Einflüsse auf Insulin können durch die Zugabe von Antioxidantien z.B. Ascorbinsäure und Tocopherol, die auch ausgezeichnet verträglich sind, reduziert werden.

Die topischen Arzneiformen mit Insulin sind kostengünstig und mit üblichen pharmazeutischen Mitteln aseptisch oder steril herstellbar. Sie gewährleisten auch gute Lagerungsstabilität als gute mikrobiologische Stabilität.

Die topischen Fertigarzneiformen mit Insulin stellen insgesamt, stufenweise abstimmbare, biochemische Zellregenerationssysteme dar. Der therapeutische Einsatz kann dabei in verschiedener Form erfolgen: Als topische Effektor-Systeme mit Insulin, als topische Effektor- Substrat- Kombinations-Systeme z.B. mit Insulin und Glukose, als synergistische Effektor- Coeffektor- Kombinationssysteme mit anderen biochemisch beteiligten Peptiden.

Auf Grund des basalen zellulären Wirkmechanismus sind die topischen Formulierungen mit Insulin therapeutisch breit einsetzbar und stellen eine Verbesserung derzeitiger therapeutischer Möglichkeiten bei problematischeren Wundheilungsverläufen oder auch in Bereichen in denen bisher keine oder eine nur unbefriedigende Therapie möglich ist. Hierzu gehören z.B. kompliziertere trophische Verhältnisse wie beim sogenannten diabetischen Fuß, bei mechanisch oder perfusionbedingtem Ulcus cruris oder bei Decubitalgeschwüren. Weiterhin können diese Formulierungen auch bei speziellen und schwieriger zu erreichenden bradytrophen Geweben eingesetzt werden, z.B. u.a. mittels topischer Applikationsformen wie intraarticulären Anwendungen bei arthrotischen Knorpelschäden. In solch einem speziellen Anwendungsfall bewirkt ein Trägervehikel auf Lipidbasis mit seinem hierin enthaltenen Wirkprinzip eine duale Verbesserung der Gelenkfunktion, sowohl durch eine mechanisch reibungsmindernde wie auch durch eine lokal metabolische Komponente der Regeneration der Knorpelschicht. Weiterhin sind auch lokale Applikationen bei Knochenfrakturen zur Verbesserung von Knochenneubildungen möglich. Auch therapeutische Regenerationen als besonders problematisch geltender Gewebsschäden, wie z.B. mechanisch oder metabolisch induzierter Nervenläsionen, sind prinzipiell anwendbar.

Im Rahmen gastrointestinaler Läsionen kann eine lokale Zellregeneration durch Kombination mit einem an der Läsion präferentiell adhäsiven Vehikel wie z.B. Sucralfat gezielt erfolgen. Die Freisetzung im geeigneten gastrointestinalen Abschnitt kann durch Wahl magensaftlöslicher bzw. magensaftunlöslicher Arzneiformen vorbestimmt werden. Die lokalen Dosierungen für proliferative und mitogene Effekte liegen weit unter denen zur Auslösung systemisch hypoglykämischer Effekte liegen. Dies konnte in verschiedenen Zellmodellen nachgewiesen werden. Somit besteht für die topischen Formulierungen auch keine Gefahr systemisch unerwünschter hypoglykämischer Wirkungen. Im Rahmen mehrerer klinischer Phase I Untersuchungen fanden sich bei gesunden menschlichen Probanden auch bei oralen Anwendungen dieser Formulierungen keine klinisch relevanten Effekte auf den Blutzucker noch auf die radioimmunologisch gemessenen Insulin-Konzentrationen im Serum.

Diabetiker zeigen gegenüber Nichtdiabetikern ein erhöhtes Wundheilungsrisiko. Da die Gewebe der Diabetiker schon insgesamt über präformierte metabolische Energiedefizite verfügen, lassen sich singuläre periphere Gewebsläsionen therapeutisch im Sinne eines regionalisiert zu behandelnden Diabetes betrachten. Die Anwendung von topischen Insulin-Formulierungen stellt daher hier eine Ergänzung der systemisch-parenteralen Insulinanwendungen dar, über die diese Läsionen nicht ausreichend erreicht werden können.

Ein Ausführungsbeispiel für die Erfindung ist nachfolgend aufgeführt, wobei dieses Beispiel die Erfindung nur weiter erläutert ohne sie hierauf zu beschränken.

Beispiel: In 100 ml einer neutralen wässrigen Insulin-Lösung mit einer Insulinkonzentration von 100 IU/ml werden 0,5% kristallines Glucose-Monohydrat gelöst. Der wässrigen Lösung wird 0,3% Aprotinin zugegeben.

In 900 ml einer flüssigen Lipidgrundlage (Topolip 492 pharmed) bestehend aus 80% einer Mischung von mittelkettigen Triacylglyceriden (50%) und mittelkettigen Partialglyceriden (30%) gesättigter Fettsäuren mit Kettenlängen von 6-18 C-Atomen sowie 20% Phosphatidycholin (Soja Lecithin) werden 0,01 % Ascorbylpalmitat und 0,05% alpha-Tocopherol eingerührt.

Die wässrige Lösung wird, unter permanenter Temperaturkontolle, bei einer Temperatur von unter 40 Grad Celsius so Lange in die Lipidgrundlage eingerührt bis sich ein klare Mikroemulsion ergibt.

Die im Beispiel aufgeführte Emulsion kann mit üblichen technischen Vorrichtungen, unter aseptischen oder Steril-Bedingungen, in anwendungsgeeignete Behältnisse, z.B. dosierbare Tropftuben aus Polymermaterial oder sonstige technisch geeignete Applikatoren direkt abgefüllt werden.

Die Emulsion wird bei 15 Grad Celsius im Kühlschrank gelagert.

Unter Bezug auf die enthaltenen Insulinkonzentrationen und die entsprechenden Spreiteigenschaften der Formulierung kann diese direkt volumendosiert auf zu behandelnde Gewebsflächen aufgetragen werden.

Zur Verbesserung und Förderung der Wundneilung und Zellregeneration werden topische Arzneiformen mit Insulin gebildet, bei denen Insulin in für lokale Anwendungszwecke pharmakologisch wirksamen Konzentrationen in pharmazeutisch für topische Anwendungen geeigneten Arzneiformen, vorzugsweise Emulsion, Suspension, Creme, Salbe, Gel oder einem Pflastersystem enthalten ist. Zur Steigerung der Wirksamkeit der topischen Anwendungsformen können als zusätzliche Bestandteile Substrate wie Einfachzucker, Cofaktoren wie Elektrolyte, andere Peptide als Insulin, Proteasehemmer und Antioxidantien zum Schutz vor Inaktivierung und Degradation, enthalten sein. Bei Emulsionen werden als Lipidphasen vorzugsweise physikochemisch und toxikologisch geeignete Mischungen von Partialgylceriden der Kettenlängen C6-C18 eingesetzt. Für interne topische Anwendungen kann der Einsatz von Vehikeln mit besonderer Wundadhäsivität erfolgen. Die topischen Arzneiformen mit Insulin sind physiologisch abgestimmte, pharmazeutisch spezifizierte topische Systeme zur Wundheilung und Zellregeneration mit biochemisch intensivierter lokaler Wirksamkeit. Sie erweitern die derzeitigen Möglichkeiten der Therapie metabolisch, physikalisch oder perfusionsbedingt verzögerter Wundheilungen und Zellregenerationen.

## Patentansprüche

1. Topische Arzneiformen mit Insulin, insbesondere zur Förderung der Wundheilung, wobei diese Insulin und Glukose in für lokale Zwecke pharmakologisch wirksamen Konzentrationen in pharmazeutisch für topische Anwendungen geeigneten Arzneiformen, vorzugsweise einer Emulsion, Suspension, Creme, Salbe, Lösung, Schüttelmixtur, einem Gel, Puder oder Granulat oder einem Pflastersystem enthalten sind, wobei diese Arzneiformen als zusätzliche Bestandteile noch andere Peptide als das Insulin, Elektrolyte, Antioxidantien enthalten können.

2. Topische Arzneiformen mit Insulin nach vorhergehendem Anspruch wobei die topischen Arzneiformen als Emulsion vorliegt, bei der Insulin in der wässrigen Phase enthalten ist und die Ölphase aus Triacylglyceriden und Partialacylglyceriden gesättigter Fettsäuren mit Kettenlängen von 6 - 18 Kohlenstoffatomen oder einer Kombination dieser Acylglyceride mit Phospholipiden, insbesondere mit Lecithin, besteht.

3. Topische Arzneiformen mit Insulin nach einem der vorhergehenden Ansprüche, wobei die topischen Arzneiformen als Vehikel polymere Stoffe enthalten, diese Stoffe insbesondere Polysaccharide und Mucopolysaccharide, Polypeptide und Proteine, Lipopolysaccharide, Glykoproteine und Lipoproteine sind und diese allein, in Kombinationen oder in Kombination mit anderen Vehikelstoffen vorliegen können.

4. Topische Arzneiformen mit Insulin nach einem der vorhergehenden Ansprüche, wobei der als zusätzlich eingesetzter Bestandteil an Einfachzucker, insbesondere Glukose ist, die als zusätzliche Cofaktoren eingesetzten Elektrolyte insbesondere Natrium, Kalium, Magnesium, Calcium und Zink oder deren Salze als Einzelstoffe oder in Kombination sind.

5. Topische Arzneiformen mit Insulin nach einem der vorher gehenden Ansprüche, wobei die zusätzlich und in Kombination mit Insulin eingesetzten Peptiden und Proteinen insbesondere sind: Platetelet Derived Growth Factor (PDGF), Platelet Derived Angiogenesis Factor (PDAF), Epidermal Growth Factor (EGF), Platelet Derived Endothelial Cell Growth Factor (PDECGF), Platelet Factor 4 (PG4), Transforming Growth Factor-β (TGF-β), Nerve Growth Factor (NGF), Insulin Like Growth Factor I und II (IGF I, IGF II), Growth Hormone (hGF), human Growth Hormone Releasing Factor (hGRF), Heparin Binding Growth factor (HBGF), Fibrocyte Growth Factor (FGF), Colony Stimulating Factor (CSF), Stretodornase, Streptokinase, und diese Stoffe als zusätzliche Einzelstoffe oder in verschiedenen Kombinationen vorliegen.

6. Topische Arzneiformen mit Insulin nach einem der vorhergehenden Ansprüche, wobei die zusätzlich enthaltenen Antioxidantien insbesondere Ascorbinsäure und Tocopherol wie auch deren Salze und Ester enthalten.

7. Verwendung einer Kombination von Insulin und Glukose zur Herstellung von topischen Arzneiformen nach einem der vorhergehenden Ansprüche für eine therapeutische Anwendung bei postoperativ, traumatisch, physikalisch oder metabolisch bedingten Wunden und Wundheilungsstörungen sowie bei topisch behandelbaren Geweben und Organen, wie bei physikalischen und biochemischen Gewebsläsionen im Bereich peripherer und zentraler Nerven, im Knochengerüst, im Bereich der Gelenke und Knorpelstrukturen, sowie im Bereich der Schleimhäute, insbesondere der Schleimhäute im Gastrointestinaltrakt und im Genitalbereich.

## Claims

1. Topical forms of medication with insulin, in particular for promoting the healing of wounds, where said insulin and glucose are contained in concentrations which are pharmacologically effective for local use in forms of medication which are pharmacologically suitable for topical applications, preferably an emulsion, suspension, creme, paste, solution, shaking mixture, gel, powder or granulate or a plaster system, and these forms of medication can contain as additional components peptides other than insulin, electrolyte, antioxidants.

2. Topical forms of medication with insulin according to the above claim, where the topical forms of medication are available as an emulsion, with insulin contained in the aqueous phase, and the oil phase is composed of triacylglycerides and partial acylglycerides saturated fatty acids with chain lengths of 6 - 18 carbon atoms or a combination of these acylglycerides with phospholipides, in particular with lecithin.

3. Topical forms of medication with insulin according to one of the above claims, where the topical forms of medication contain vehicles in the form of polymer materials, these materials are in particular polysaccharides and mucopolysaccharides, polypeptides and proteins, lipopolysaccharides, glycoproteins and lipoproteins, and these can be present on their own, in combinations or in combination with other vehicle materials.

4. Topical forms of medication with insulin according to one of the above claims, where an additionally applied component is simple sugar, in particular glucose, and electrolytes applied as additional cofactors are in particular sodium, potassium, magnesium, calcium and zinc or their salts as individual materials or in combination.

5. Topical forms of medication with insulin according to one of the above claims, where peptides and proteins applied additionally and in combination with insulin are in particular: Platelet Derived Growth Factor (PDGF), Platelet Derived Angiogenesis Factor (PDAF), Epidermal Growth Factor (EGF), Platelet Derived Endothelial Cell Growth Factor (PDECGF), Platelet Factor 4 (PG4), Transforming Growth Factor-β (TGF-β), Nerve Growth Factor (NGF), Insulinlike Growth Factor I and II (IGF I, IGF II), Growth Hormone (hGF), human Growth Hormone Releasing Factor (hGRF), Heparin Binding Growth Factor (HBGF), Fibrocyte Growth Factor (FGF), Colony Stimulating Factor (CSF), Streptodornase, Streptokinase, and these materials are present as additional individual materials or in different combinations.

6. Topical forms of medication with insulin according to one of the above claims, where the additional contained antioxidants contain in particular ascorbic acid and tocopherol as well as their salts and esters.

7. Use of a combination of insulin and glucose for manufacturing topical forms of medication according to one of the above claims for a therapeutical application with post-operative, traumatic, physically or metabolically based wounds and interferences in wound healing as well as topically treatable tissues and organs, as in physical and biochemical tissue lesions in the area of peripheral and central nerves, in the bone structure, in the area of the joints and cartilage structures as well as in the area of mucosae, in particular mucosae in the gastrointestinal tract and in the genital area.

## Revendications

1. Formes galéniques à usage topique contenant de l'insuline, en particulier pour l'accélération de la cicatrisation, l'insuline et du glucose étant contenus en des concentrations pharmacologiquement efficaces à des fins locales dans des formes galéniques pharmaceutiquement appropriées à des applications topiques, de préférence une émulsion, une suspension, une crème, une pommade, une solution, une mixture à agiter, un gel, une poudre ou des granulés ou un système d'emplâtre, ces formes galéniques pouvant contenir, comme constituants supplémentaires, encore d'autres peptides différents de l'insuline, des électrolytes et des anti-oxydants.

2. Formes galéniques à usage topique contenant de l'insuline selon la revendication précédente, lesdites formes galéniques à usage topique se présentant sous la forme d'une émulsion dans laquelle de l'insuline est contenue dans la phase aqueuse, et la phase huileuse est formée de triacylglycérides et d'acylglycérides partiels d'acides gras saturés ayant des longueurs de chaîne de 6 à 18 atomes de carbone ou d'une combinaison de ces acylglycérides avec des phospholipides, en particulier avec de la lécithine.

3. Formes galéniques à usage topique contenant de l'insuline selon l'une des revendications précédentes, lesdites formes galéniques à usage topique contenant des substances polymères en tant que véhicules, ces substances étant en particulier des polysaccharides et des mucopolysaccharides, des polypeptides et des protéines, des lipopolysaccharides, des glycoprotéines et des lipoprotéines, et celles-ci peuvent être présentes individuellement, sous la forme de combinaisons entre elles ou sous la forme d'une combinaison avec d'autres véhicules.

4. Formes galéniques à usage topique contenant de l'insuline selon l'une des revendications précédentes, dans lesquelles le constituant utilisé de façon supplémentaire est un sucre simple, en particulier du glucose, les électrolytes utilisés en tant que co-facteurs supplémentaires sont en particulier du sodium, du potassium, du magnésium, du calcium et du zinc, ou des sels de ceux-ci, sous la forme de substances individuelles ou combinées.

5. Formes galéniques à usage topique contenant de l'insuline selon l'une des revendications précédentes, dans lesquelles les peptides et les protéines utilisés de façon supplémentaire et combinés avec l'insuline sont en particulier : le facteur de croissance dérivé des plaquettes (PDGF), le facteur d'angiogenèse dérivé des plaquettes (PDAF), le facteur de croissance épidermique (EGF), le facteur de croissance des cellules endothéliales dérivé des plaquettes (PDECGF), le facteur plaquettaire 4 (PF4), le facteur de croissance transformant β (TGF-β), le facteur de croissance nerveuse (NGF), le facteur de croissance analogue à l'insuline I et II (IGF I, IGF II), l'hormone de croissance (hGF), le facteur déclenchant la sécrétion de l'hormone de croissance humaine (hGRF), le facteur de croissance fixant l'héparine (HBGF), le facteur de croissance des fibrocytes, le facteur stimulant la formation de colonies (CSF), la streptodornase, la streptokinase, et ces substances se présentent sous la forme de substances supplémentaires individuelles ou diversement combinées.

6. Formes galéniques à usage topique contenant de l'insuline selon l'une des revendications précédentes, dans lesquelles les anti-oxydants contenus de façon supplémentaire contiennent en particulier de l'acide ascorbique et du tocophérol, ainsi que leurs sels et leurs esters.

7. Utilisation d'une combinaison d'insuline et de glucose pour la préparation de formes galéniques à usage topique selon l'une des revendications précédentes pour une application thérapeutique dans le cas de blessures d'origine post-opératoire, traumatique, physique ou métabolique et de troubles de la cicatrisation, ainsi que pour des tissus et des organes pouvant être traités, comme des lésions tissulaires physiques et biochimiques dans la région des nerfs périphériques et centraux, dans l'ossature, dans la région des articulations et des structures cartilagineuses, ainsi que dans la région des muqueuses, en particulier des muqueuses de l'appareil gastro-intestinal et de l'appareil génital.
